# EUROPEAN PATENT SPECIFICATION

(11) **EP 2 189 149 B1**
(45) Date of publication and mention of the grant of the patent: **13.12.2017**
(21) Application number: 09155203.4
(22) Date of filing: 16.03.2009
(51) Int. Cl.: A61K 8/04, A61K 8/26, A61K 8/92, A61Q 15/00, A61K 8/25, A61K 8/34, A61K 8/02

(54) **Antiperspirant compositions**
Schweißhemmende Zusammensetzungen
Compositions anti-transpirantes

(30) Priority: 24.11.2008 US 117288
(43) Date of publication of application: 26.05.2010
(73) Proprietor: Unilever PLC, A Company Registered in England and Wales under Company no. 41424, London EC4Y 0DY Greater London (GB); Unilever N.V., 3013 AL Rotterdam (NL)
(72) Inventor: Butterworth, Andrew, Leeds, LS14 2AR (GB); Ferrier, Lindsay Karen, Leeds, LS14 2AR (GB); Fletcher, Neil Robert, Bebington, Wirral, Merseyside CXH63 3JW (GB); Jones, Shirley, Seacroft, Leeds, LS14 2AR (GB); Marriott, Robert Edward, Bebington, Wirral, Meerseyside CH63 3JW (GB); Polonka, Jack, Trumbull, Connecticut 06611 (US); Williams, Jason Richard, Bebington, Wirral, Merseyside CH63 3JW (GB)
(74) Representative: Whaley, Christopher

(56) References cited:
- EP-A- 0 334 203
- EP-A- 1 428 521
- WO-A-2005/048966
- WO-A-2005/074877
- WO-A1-2007/031137
- GB-A- 2 299 024
- US-A- 3 923 971
- US-A- 4 889 711
- US-B1- 6 375 938
- ANONYMOUS: "MIRASIL WAX-B" INTERNET ARTICLE, [Online] XP002537447 Retrieved from the Internet: URL:http://www.delfino.com/rhodiapc/text_a nd_graphics/pdf/Mirasil_WAX_B.pdf> [retrieved on 2009-07-15]
- ANONYMOUS: "Sonneborn - White Oils, Petrolatums, Petroleum Distillates, Microcrystalline Waxes" INTERNET ARTICLE, [Online] XP002537448 Retrieved from the Internet: URL:http://www.sonneborn.com/products/pdf/ sonneborn_brochure.pdf> [retrieved on 2009-07-16]
- DATABASE CA [Online] CHEMICAL ABSTRACTS SERVICE, COLUMBUS, OHIO, US; 2005, Nishihama, Shuji: "Special functions of clay minerals in cosmetics; Noble makeup products using TiO2 coated mica", Database accession no. 142:416752

## Description

The present invention relates to antiperspirant compositions and particularly to aerosol antiperspirant compositions and to cosmetic methods of controlling perspiration from localised areas of the body, such as from the underarm.

### Background and Prior Art

For many years, humans have employed cosmetic methods, sometimes alternatively referred to as non-therapeutic methods, to prevent or at least ameliorate bodily functions which local society at the time considers to be unsightly or otherwise undesirable. These methods have included controlling the appearance of sweat by topical application of an active ingredient which prevents egress of sweat from the eccrine glands. The active can be applied cosmetically and topically to the skin, broadly speaking, by one of two methods. Different consumers prefer one method or the other. In one method, sometimes called a contact method, a composition is wiped across the surface of the skin, depositing a fraction of the composition as it passes. In the second method, sometimes called the non-contact method, the composition is sprayed from a dispenser held proximate to the skin, often in the region of 10 to 20cms. The spray can be developed by mechanical means of generating pressure on the contents of the dispenser, such as a pump or a squeezable sidewall or by internally generated pressure arising from a fraction of a liquefied propellant volatilising, the dispenser commonly being called an aerosol.

In cosmetic aerosol compositions, the antiperspirant active is commonly an aluminium active. An aerosol dispenser has to withstand the internal pressure generated by the propellant so that it is usually made from metal. Aerosol compositions are commonly anhydrous, because the aluminium antiperspirant active would dissolve in water-containing formulations generating an acidic solution that can cause or exacerbate corrosion of metal canisters.

Cosmetic products are employed by consumers in order to improve their appearance, and although for many years antiperspirant compositions in general and aluminium-containing antiperspirant aerosol compositions have been employed primarily to reduce visible wetness on various regions of the body, and especially in the underarm, some consumers are seeking even further benefits. A substantial number of female users of underarm antiperspirants shave their armpits, or pluck the hairs that grow there, an act which is considered by parts of society to improve their appearance. Shaving and plucking irritates human skin resulting often in localised reddening, which can manifest itself as blotchiness. The antiperspirant active itself can likewise irritate the skin by demoisturisation in between the eccrine glands and the two actions together combine to disfigure the armpit. Also, as has been long since recognised, an aluminium antiperspirant, and especially a particulate aluminium antiperspirant, tends to deposit a visible whiteness on the skin. Furthermore, as a person becomes older, skin progressively loses its inherent elasticity and becomes rougher and more wrinkled, even skin in the underarm that is typically often covered by clothing or least is usually out of the direct glare of harmful UV radiation. Moreover, the very materials that are necessary to inhibit localised perspiration, themselves tend to demoisturise the skin, reducing its elasticity and can even cause increased skin irritation, impairing the visual appearance of skin as well as its physical properties. Although a moisturising agent or agents has been incorporated in Dove antiperspirant compositions to counteract or ameliorate the impairment of skin elasticity, such as the incorporation of an occlusive agent, it has relatively little visual benefit. WO 2007/031137 A1 relates to anhydrous antiperspirant spray compositions which reduce roughness of the skin and improve the appearance of fine lines/wrinkles. To summarise, a need has been identified to counteract the impairment in visual appearance of underarm skin itself arising from performance of common acts to enhance beauty, such as shaving together with applying an effective but non-therapeutic antiperspirant to counteract perspiration.

In the course of conducting investigations into how to improve the beauty of underarm skin whilst simultaneously applying a cosmetic antiperspirant composition, the team including the instant inventors recognised that the incorporation of a smoothness aid could assist, provided that it was chosen appropriately. A number of factors needed to be balanced or taken into account.

First, in order to remain useable as an aerosol, the viscosity of the composition had to be restrained to permit it to be sprayed through conventional dispensing apparatus. Secondly, the composition after its application and volatilisation of the propellant, should offer a pleasing appearance to the eye in such respects as tone, radiance and smoothness and not to exhibit excessive visible whiteness.

By way of reference, a commercial antiperspirant aerosol product (Dove) has been sold for several years which comprised a triglyceride emollient oil together with a moisturiser (an occlusive agent) in order to retain moisture and thereby enhance skin elasticity. Whilst this achieved its intended result, and helped to counteract shaving or plucking, it was comparatively ineffective at concealing skin imperfections, or improving the tone, radiance and smoothness of underarm skin. A number of materials were tried that had been suggested or incorporated in skin-care products that were not anhydrous antiperspirant aerosols. Such materials included polymethylmethacrylate and silicone elastomers. Regrettably, they did not achieve the desired improvement in skin tone, radiance and/or smoothness when employed at a concentration that would have been desirable in aerosol compositions.

It is an object of the present invention to identify a smoothing aid for an aerosol antiperspirant composition that improves the beauty of underarm skin or at last ameliorates the effect of employing the antiperspirant itself or the effect of underarm challenges such as shaving or plucking of hair.

### Brief summary of the present invention

According to one aspect of the present invention, there is provided an anhydrous antiperspirant composition according to claim 1. It has been found that by incorporating a wax in an aerosol composition, it is possible to improve the visible appearance of armpit skin, such as its tone, imperfections, smoothness and/or radiance. It is not necessary to employ a high concentration of the wax, and indeed, it is possible to achieve a desired outcome at a concentration less than that at which the composition would be thickened to such an extent that could not be readily sprayed or would suffer a significant risk of blocking the spray outlet in use. Without being bound by any theory, hypothesis or conjecture, it is believed that when the composition containing the wax is sprayed onto human skin, the wax, or at least some of it, may form small globules that can act to refract or scatter light, thereby diffusing the appearance of the skin on which the wax has been deposited. By way of analogy, the diffusion can be compared with viewing an object through frosted glass - the object is visible but with a fuzzy outline.

In a second aspect of the invention, there is provided a means for enhancing the appearance of skin to which an astringent aluminium salt has been applied topically from an anhydrous aerosol composition as an antiperspirant which further comprises a selected smoothing aid.

In a third aspect of the present invention, there is provided a means for enhancing the appearance of skin to which an astringent aluminium salt has been applied topically from an anhydrous aerosol composition in which its base formulation contains an astringent aluminium salt that acts as an antiperspirant and a selected smoothing aid together with one or more moisturisers and/or a triglyceride oil.

In a further aspect of the present invention there is provided a method for mitigating the impaired visual appearance of skin, such as underarm skin, arising from topical application thereon of an aluminium-containing astringent antiperspirant salt by simultaneous spraying of a moisturiser and/or a triglyceride oil together with a wax and/or mica.

Other and further aspects of the present invention may be apparent from the subsequent text herein.

Detailed description of the present invention and preferred embodiments thereof.

The present invention in certain aspects contemplates incorporating a small concentration of wax in an anhydrous aerosol containing an astringent aluminium salt as an antiperspirant. The concentration of the wax is insufficient to solidify the base formulation.

The aerosol compositions herein comprise a base formulation which is mixed with a liquefiable propellant gas such that at the pressure in the dispenser, the propellant is a liquid or with a propellant that remains gaseous at elevated pressure.

A concentration of an ingredient given herein is based by weight on the base formulation, unless expressly specified otherwise.

The present invention seeks to ameliorate one or more visual impairments of underarm skin and/or improve positive attributes such as tone, smoothness and/or radiance, and thereby render the skin more attractive by creating a soft focus effect.

Herein, soft-focus effect of the film of antiperspirant composition on the skin applied by spraying is indicative of how strongly it scatters light. The soft-focus measurement is obtained by dividing the diffuse transmittance of the film by the total transmittance of a layer of that film.

Opacity is the degree to which the film of antiperspirant composition visibly covers the skin upon which it has been applied. The opacity is determined by measuring the diffuse reflectance of a film of similar thickness of the antiperspirant composition on a black substrate and dividing it by the diffuse reflectance of the film on a white substrate.

Gloss indicates shine in relation to the film. Herein, its gloss of the film is its reflectance measured at the specular angle of 60° from the normal to the film surface.

The instant invention in at least some preferred embodiments balances matt and gloss effects.

### Base Formulation

### Particulate Antiperspirant Active

Herein, the antiperspirant active is a particulate astringent aluminium salt and particularly a basic salt such as a basic aluminium salt. It is especially desirable to employ an aluminium chlorohydrate, by which is meant herein a material which satisfies the empirical formula Al₂(OH)ₓCl_{y} in which x + y = 6 and y is normally at least 0.5 and commonly not greater than 1.8, the material usually comprising bound water of hydration. The weight proportion of said water of hydration is conventionally not more than 12% and often lies in the range of from 3 to 10%. The term aluminium chlorohydrate herein encompasses materials with specified figures for x and y, such as aluminium sesquichlorohydrate and materials in which the chlorohydrate is present as a complex. It will be recognised that alternative names are sometimes used to indicate the presence of hydroxyl substitution, including aluminium hydroxychloride, aluminium oxychloride or basic aluminium chloride.

Aluminium chlorohydrate, as made, comprises a mixture of a number of different polymeric species in varying proportions, depending on the molar ratio of aluminium to chloride and the conditions employed during manufacture. All such mixtures are employable herein. It is especially desirable to employ what is commonly called activated aluminium chlorohydrate or enhanced activity aluminium chlorohydrate, sometimes abbreviated to AACH, in which the proportion of the more active species is higher by virtue of its method of manufacture. In one definition of activated, given in EP 6739, the material has greater than 20% Band III. Other methods of making AACH are given in EP 191628 and EP 451395. AACH is often made by recovery of an aluminium chlorohydrate from a dilute solution under strictly controlled reaction/maturing/dewatering/drying conditions. AACH is commercially available by name from suppliers such as Reheis, and B K Giulini.

Aluminium chlorohydrate, whether it be activated or not activated, can be complexed, whereupon the CTFA name for the complex is concatenated to aluminium chlorhydrex, followed by the name of the molecule with which it is complexed. Commonly, such complexes include propylene glycol, that is representative of C₂ to C₆ glycols and glycine, that is representative of aminoacids. Any such complexed molecules remain complexed during formation of the invention base and aerosol compositions herein.

The particles of the astringent salt employed herein, such as an aluminium chlorohydrate, be it activated, complexed or otherwise, desirably have a diameter of below 125 microns, preferably ≥ 99% by weight below 100 µm and especially ≥ 95% by weight below 75 µm. Commonly, the astringent salt herein has a weight average mean particle size selected in the range of from 10 to 30 µm. In some embodiments, said mean is from 13 to 18 µm and in other embodiments, said mean is from 18 to 27µm. In a number of particularly preferred embodiments, the astringent salt is an activated aluminium chlorohydrate having said mean of from 14 to 26 µm.

Herein, unless otherwise stated, particle sizes (diameters) and distributions for the antiperspirant active are those that are obtainable by laser light scattering, for example obtained from the appropriate Mastersizer instrument for anhydrous suspensions, obtainable from Malvern Instruments set to produce a volume plot. The instrument is employed with a lens selected in accordance with the maker's instructions to accommodate the expected particle size distribution, (or various lenses can be tested until the best lens is identified) and is preferably operated employing cyclomethicone (DC245™ from Dow Corning) as the liquid dispersant for a sample of the base formulation to attain a particles concentration that achieves obscuration, i.e. 10-30% light scattered. Using the Polydisperse analysis model and knowing the dispersant RI, the RI of the particulate material and imaginary RI factor of 0.1, the plot of the particles size (d) distribution and the average particle size D50 is obtained.

The weight proportion of antiperspirant salt, eg aluminium chlorohydrate of AACH in the base formulation is commonly selected in the range of from 5 to 60%, often not greater than 50% and in many suitable embodiments from 30 to 45% by weight, the weight including any water of hydration and complexed molecules. The proportion of such active in the base formulation is often selected in conjunction with the weight ratio of propellant to base formulation such that the weight proportion of aluminium salt in the full aerosol composition is from 1 to 15% and particularly from 2 to 12%.

### Oil

The base formulation comprises an oil or preferably a mixture of oils in which particulate materials are suspended, so that the aerosol expels a pattern of liquid droplets when sprayed. The term oil herein indicates a hydrophobic material that is liquid at 20°C at 1 atmosphere pressure. It will be recognised that various of the oils contemplated herein can provide one or more functions in addition to acting as a carrier for particulate materials, for example some act as an emollient, or mask skin deposits to alter the appearance of the aerosol composition when applied topically, and/or some can mask odour of the composition itself or malodours generated on skin from secretions.

The proportion carrier fluids in the base formulation, including oils and optional or other functional ingredients which are liquid at 20°C and oil-miscible, is often at least 35%, and in many base formulations at least 45%, is commonly not higher than 85%, in many embodiments not higher than 75% and in several practical formulations not higher than 65%, %s being by weight of the base formulation. In a number of highly desirable base formulations, the oil weight proportion is from 45 to 85%, particularly 45 to 70%, and in certain desirable base formulations is from 50 to 60%. The term carrier fluid is employed in regard to the base formulation and therefore excludes propellant which is subsequently mixed with the base formulation to create the aerosol composition.

The oils employed in the instant invention often desirably comprise one or more volatile silicone oils. By volatile herein is meant having a measurable vapour pressure at 20 or 25^{□}C of at least 1 Pa. Typically the vapour pressure of a volatile silicone oil lies in a range from 1 or 10 Pa to 2 kPa at 25^{□}C. Volatile silicone oils can be linear or cyclic siloxanes, usually containing from 3 to 9 silicon atoms, and commonly from 4 to 6 silicon atoms, the silicon atoms being substituted by methyl groups, so that their alternative names are methicones and cyclomethicones. It is especially desirable to employ volatile silicone oils in which at least 80% by weight and particularly at least 90% contain at least 5 silicon atoms, such as cyclopentadimethylsiloxane (D5), cyclohexadimethylsiloxane (D6), dodecamethylpentasiloxane and tetradecamethylhexasiloxane. The cylomethicone oils are especially preferred. Such oils are highly desirable for many consumers because they can evaporate without causing undue skin cooling. The volatile silicone oils often comprise at least 30% by weight of the carriers fluids and normally not higher than 95% thereof, and in a number of desirable formulations the weight proportion of the carrier fluids is at least 35%, and particularly at least 40% and in the same or other embodiments of the invention is up to 75%, especially up to 65% and particularly up to 55%.

The oils in the carrier fluid preferably comprise one or more non-volatile oils, which can be silicone oils and/or particularly non-silicone oils. Preferably, in some embodiments at least a fraction of the non-volatile oils has a refractive index of at least 1.45. Such oils in the base formulation can advantageously lessen the appearance of visible residues on skin, only immediately on application but also throughout the period typically from 6 to 24 hours before the antiperspirant formulation is washed off.

Non-volatile silicone oils employed herein preferably contain one or more unsaturated substituents such as phenyl or diphenylethyl in replacement of the corresponding number of methyl substituents in polycyclosiloxanes or more preferably in linear siloxanes, often having 2 or 3 silicon atoms. Such non-volatile oils have a higher refractive index than that of the volatile silicone oils and tend to mask the antiperspirant active when it is deposited on skin. The non-volatile oils can also comprise dimethiconols, which as the name suggests are hydroxyl-terminated. The proportion of non-volatile silicone oils in the carrier fluids is conveniently from 0 or 0.25 to 10% by weight and often from 0.5 to 5% by weight, such as in the range of from 1 to 3% by weight of the oils.

The oils in the carrier fluid can alternatively or additionally comprise one or more hydrocarbon fluids (oils), which can be either volatile or non-volatile. Suitable hydrocarbon fluids include liquid aliphatic hydrocarbons such as mineral oils or hydrogenated polyisobutene, desirably selected to exhibit a low viscosity. Further examples of liquid hydrocarbons are polydecene and paraffins and isoparaffins of at least 10 carbon atoms. Hydrocarbon fluids conveniently comprise from 0 to 25% by weight of the carrier.

In at least some advantageous embodiments, the carrier oils comprise liquid aliphatic or aromatic ester oils. Suitable aliphatic esters contain at least one long chain alkyl group, such as esters derived from C₁ to C₂₀ alkanols esterified with a C₈ to C₂₂ alkanoic acid or C₆ to C₁₀ alkanedioic acid. The alkanol and acid moieties or mixtures thereof are preferably selected such that they each have a melting point of below 20°C. Aliphatic esters include isopropyl myristate, lauryl myristate, isopropyl palmitate, diisopropyl sebacate and diisopropyl adipate. Further and very suitable ester oils include glyceride oils and in particular triglyceride oils derived from glycerol and fatty acids containing at least 6 carbons and especially natural oils. The weight proportion of aliphatic esters in the base formulation is often up to 10%, such as at least 1%.

It is particularly desirable to include a liquid aromatic ester include fatty alkyl benzoates. Examples of such esters include suitable C₈ to C₁₈ alkyl benzoates or mixtures thereof, including in particular C₁₂ to C₁₅ alkyl benzoates eg those available under the trademark Finsolv. An aryl benzoate, such as benzyl benzoate can also be used. Yet other suitable ester oils includes oils in which a short alkylene group of 1 to 3 carbons, optionally substituted by a methyl group, is interposed between benzene and benzoate residues. The weight proportion of such aromatic ester oils is often from 2.5 to 10% in the base formulation and particularly from 3.5 to 7%.

The total proportion of ester oils, including both aliphatic and aromatic ester oils (but excluding any fragrance oil) is often from 0 to 50% by weight of the oil mixture, is desirably at least 2% and especially is at least 5% by weight. Their total proportion is in many embodiments desirably up to 30% and particularly attractively up to 20% by weight of the oil mixture. In some especially desirable formulations, the ester oils constitute from 7.5 to 20% of the oils mixture. Expressed on the basis of the base formulation, the ester oils often constitute from 3.5 to 16% by weight and particularly from 4.5 to 13% by weight. The weight ratio of aromatic ester oil to aliphatic ester oil is often selected in the range of from 1:3 to 10:1 and especially from 1:2 to 2:1.

Natural oils which are most desirably employed herein comprise triglyceride oils such as in particular one or more unsaturated C₁₈ fatty acid glycerides. In many instances, the oils comprise one or more triglycerides. The fatty acid residues in the oils can comprise, commonly, from one to three olefinic unsaturated bonds and often one or two. Whilst in many instances the olefinic bonds adopt the trans configuration, in a number of desirable products the bond or bonds adopt the cis configuration. If two or three olefinic unsaturated bonds are present, they can be conjugated. The fatty acid can also be substituted by an hydroxyl group. The natural oils employable herein desirably comprise one or more triglycerides of oleic acid, linoleic acid, conjugated linoleic acid, linolenic acid or ricinoleic acid. Various isomers of such acids often have common names, including linolenelaidic acid, trans 7-octadecenoic acid, parinaric acid, pinolenic acid punicic acid, petroselenic acid and stearidonic acid. It is especially desirable to employ glycerides derived from oleic acid, linoleic acid or petroselenic acid, or a mixture containing one or more of them.

Natural oils containing one or more of such triglycerides include coriander seed oil for derivatives of petroselinic acid, impatiens balsimina seed oil, parinarium laurinarium kernel fat or sabastiana brasilinensis seed oil for derivatives of cis-parinaric acid, dehydrated castor seed oil, for derivatives of conjugated linoleic acids, borage seed oil and evening primrose oil for derivatives of linoleic and linolenic acids, aquilegia vulgaris oil for columbinic acid and sunflower oil, olive oil or safflower oil for derivatives of oleic acid, often together with linoleic acids. Other suitable oils are obtainable from hemp, which can be processed to derive stearadonic acid derivatives and maize corn oil. An especially convenient natural oil by virtue of its characteristics and availability comprises sunflower oil, ranging from those rich in oleic acid glycerides to those rich in linoleic acid glycerides, rich indicating that its content is higher than that of the other named acid.

The proportion of the natural oil, viz particularly the triglyceride oils of unsaturated fatty acids, in the formulation is often selected in the range of from 0.1 to 15% by weight of the oils mixture, desirably at least 0.25% by weight, especially in the range of from at least 0.5 of the oils mixture.

Expressed as a proportion of the base formulation, in some beneficial embodiments, the triglyceride oil represents from 1 to 8% by weight, particularly from 2 to 6% and advantageously from 3 to 5% by weight thereof.

Additionally or alternatively, the base formulation can comprise jojoba oil such as in a proportion of up to 5% w/w of the base formulation.

A further class of particularly suitable carrier oils comprise non-volatile liquid aliphatic ethers derived from at least one fatty alcohol that desirably contains at least 10 carbon atoms, such as myristyl ether derivatives e.g. PPG-3 myristyl ether or lower alkyl (≤ C₆) ethers of polygylcols (preferably polypropylene glycol and especially 10 to 20 units, such as an ether named as PPG-14 butyl ether in the CTFA. It is often convenient for the aliphatic ether to constitute at least 10%, and especially at least 15%, particularly up to 60% and very desirably up to 50% by weight of the oils mixture, including the range of from 30 to 45%. Expressed as a weight proportion of the base formulation, the ether oil constitute desirably from 15 to 30% and particularly from 20 to 25% by weight.

Such ethers, and especially those having a refractive index of above 1.46 can assist in masking the visibility of deposits on the skin, thereby complementing the positive skin conditioning properties of the overall formulation. It can be very desirable to select the ether in a weight ratio to the antiperspirant active of at least 0.3:1 such as up to 0.8:1, one practical range being from 0.5:1 to 0.7:1.

It is often convenient to select the aromatic ester oil plus non-volatile aliphatic ether to volatile silicone oil in a weight ratio of from 3:2 to 3:1, and especially from 1.75:1 to 2.5:1.

A further class of oils can be employed beneficially herein comprises water-immiscible aliphatic alcohols, and particularly those having a boiling point of higher than 100°C. These include branched chain alcohols of at least 10 carbon atoms and in many instances up to 30 carbon atoms, particularly 15 to 25, such as isostearyl alcohol, hexyl-decanol and octyl-dodecanol. It will be recognised that octyl dodecanol is an especially favoured water-immiscible aliphatic alcohol in the instant formulations, because it not only acts as an emollient oil but additionally moisturises skin by the mechanism of occlusion. Such alcohols can often constitute at least 0.1% and particularly at least 0.5% by weight of the oils mixture, and in many formulations are not more than 5% and particularly up to 3% by weight of the oils mixture. Expressed as a weight proportion of the base formulation, the branched alcohol desirable comprises from 0.25 to 2% by weight thereof, and particularly from 0.5 to 1.5% thereof. In some especially desirable embodiments, the branched fatty alcohol is present in a weight ratio to the smoothing aids of from 1:2 to 2:1.

In the invention base formulations, the total weight proportion of non-volatile oils is often falling in the range of from 30 to 50% and particularly from 35 to 45%. The total weight proportion of volatile oils in the invention base formulation is often chosen in the range of from 12.5 to 25%, and in many very suitable embodiments from 15 to 22.5%. The weight ratio of non-volatile to volatile oils is desirably chosen in the range of from 2:1 to 4:1 , particularly from 3:2 to 5:2 and especially from 7:4 to 9:4.

### Smoothing Aid

The base formulation of the aerosol compositions disclosed herein contains at least one smoothing aid, of which two classes are disclosed in more detail. One class comprises a wax and the other class comprises mica. Again without being bound by theory, mica is highly desirable because it can be considered to adopt the form of platelets. As such, it is a preferred representative of a broader class of minerals comprising alumino-silicate minerals that likewise adopt the form of platelets. The anhydrous antiperspirant composition comprises both a wax and mica. One tends to provide a matt finish, whereas the other increases reflectance, so that the two together enhance the soft-focus appearance of skin onto which has been sprayed the thin film of antiperspirant composition.

Without being bound by theory as to why the soft focus of the skin is improved, incorporation of a limited concentration of wax is observed to offer a matt finish to the antiperspirant film. The waxes that can be contemplated for employment herein normally have a melting point of up to 90°C and often up to 80°C. Typically, desirable waxes have a melting point of at least 50°C, and especially at least 55°C. A number of highly desirable waxes melt in the range of up to about 70°C. The waxes are selected from:-
hydrocarbon waxes, such as mineral waxes or polyethylene, commonly having an average molecular weight of from 250 to 600;
aliphatic ester waxes, such as esters derived from either or both of a fatty acid or alcohol, the resultant ester often containing from 18 to 40 carbon atoms; natural waxes derived from plants or animals, such as beeswax, candelilla wax and caster wax
and, preferably, linear fatty alcohol waxes, commonly containing from 14 to 22 carbon atoms, such as cetyl alcohol, stearyl alcohol, or eicosyl alcohol.

The waxes can be natural or synthesised. Mixtures of two or more waxes can be employed, including mixtures from different subclasses. The wax comprises stearyl alcohol or a mixture of waxes comprising stearyl alcohol, such as cetearyl alcohol.

When selecting the wax or waxes to employ, it is preferred to employ a wax that is amorphous rather than crystalline, and particularly effective results have been achieved from a wax that is not only amorphous, but also has a comparatively low melting point, such as in the range of from 55 to 65°C.

A low concentration of wax, and especially an amorphous wax, in the base formulation is deliberately chosen. It will be recognised that to at least some extent, during processing of the base formulation and the aerosol composition, intensive mixing operations can result in localised heat generation, leading to dissolution at least a fraction of the wax into the carrier oil (or blend of oil with the propellant, as the case may be). Localised melting of the wax can possibly also contemplate wax droplet formation.

As the concentration of wax increases, there is a tendency for the resultant aerosol composition to exhibit a higher viscosity, making it more difficult for the composition to be sprayed from the aerosol container and increasing the risk of blockage of the spray outlet (the spray nozzle) and/or the outlet control valve. Viscosity increase can also be compounded by the risk of particulates agglomeration as the proportion of wax increases in the base formulation.

Thus, in practice, there is a concentration window for the wax that takes into account both the desirability of providing a smoothing aid and the desirability of not increasing the viscosity of the composition too much. The wax is present in the base formulation at a concentration of up to 1.5% by weight. The wax is present at a concentration of at least 0.4% by weight. In the invention compositions contemplated herein, the weight ratio of total liquid to wax in the aerosol formulation, i.e. both liquids in the base formulation plus the propellant, is desirably greater than 140:1, and in many practical embodiments is greater than 200:1 such as in the range of from 250:1 up to 2000:1, and particularly from 500:1 to 1250:1.

The above limits for the concentration of the wax and its ratio to liquids in the base formulation are particularly suitable when the proportion of non-volatile oil in the base formulation comprises from 35 to 70% of the total of liquids in the base formulation.

The second class of smoothing aids contemplated herein comprises in particular mica, although may alternatively be considered to include other alumino-silicate minerals that are deemed to adopt the form of platelets. The mica desirably has a weight-averaged mean particle size of below 50µm and especially up to 30 µm. If desired more than one mica material can be employed. For practical reasons, the weight-averaged mean particle size of at least one mica material employed is preferably at least 7µm and in some advantageous embodiments is at least 15 µm. One convenient combination of mica materials comprises one material with - averaged mean particle size of 7 to 14 microns and a second material with weight-averaged mean particle size of 18 to 25 microns. In many desirable embodiments herein, the weight average mean particle size of the mica and the aluminium antiperspirant salt are both selected in the range of from 7 to 30 microns and are present in a ratio of from 2:1 to 1:2.

In some or other embodiments, herein, the mica can be employed without further treatment. In other and much favoured embodiments, the mica has been surface treated with titanium dioxide, and/or tin oxide and/or silicon dioxide, thereby forming a mica pigment. In particularly favoured embodiments of the present invention, the mica surface treatment further incorporates a transition metal oxide imparting a colour, so that the resultant material is an interference pigment. Suitable transition metal oxides include iron and chromium oxide. By the choice of the complementary oxide, the producer of the pigment is able to create pigments exhibiting the full visible spectrum of colour highlights, as well as silver. It is especially suitable to select mica interference pigments having blue, green or violet highlights, i.e. a wavelength of less than about 550nm, and particularly less than 500nm. The interference pigment constitutes a fraction of the mica pigment, such as from 20 to 75% by weight. The mica, including a mica pigment, is employed in conjunction with the above-mentioned wax. In other embodiments, which can provide at last some of the benefit arising from use of a combination of wax plus mica, the smoothing aid is a mica pigment exhibiting highlights of wavelength less than about 550 nm and particularly less than 500 nm.

The weight proportion of mica in the invention compositions herein is at least 0.1 % of the base formulation and preferably at least 0.25%. Its weight proportion is normally not greater than 5% and commonly not greater than 2%. When employed together with a wax, the weight ratio is often from 3:1 to 1:3, and in many practical embodiments from 2:1 to 1:2.

In a number of embodiments in accordance with the third aspect of the present invention, there is provided an anhydrous aerosol antiperspirant composition in which the base formulation comprises the smoothing aid comprising mica or/and wax in a concentration of at least 0.25%, together with a moisturiser at a concentration of at least 0.25% by weight and/or a triglyceride oil at a concentration of at least 1 % by weight. Preferred smoothing aids, antiperspirant actives and triglyceride oils, and combinations thereof, as well as preferred amounts are described herein in greater detail.

The moisturiser for employment in conjunction with the third aspect can suitably be an occlusive agent, such as an occlusive oil, e.g. octyldodecanol. It will be recognised that as a general rule, most water-immiscible ester oils, such as isopropyl myristate or alkyl C₁₂₋₁₅ benzoate act as an emollient oil, but do not act as an occlusive agent. The moisturising agent in the third aspect can alternatively comprise polyethylene glycol (PEG) desirably having an average molecular weight of from 200 to 625 and particularly from 250 to 450, advantageously in a concentration of from 1 to 5% by weight of the base formulation.

In addition to the antiperspirant salt, the oil and the smoothing aid or aids, the base formulation preferably contains a particulate suspending aid. Suitable suspending aids include colloidal silicas, suitably pyrogenic silica, and clays such as montmorillonite clays such as bentonite and hectorite. Particularly desirably, the suspending aids have a hydrophobically treated surface. A particularly preferred bentonite is hydrophobic bentonite, e.g. aids which are commercially available under the trade mark Bentone, eg Bentone LT, Bentone 14, Bentone 27, Bentone 34, and Bentone 38 / 38V and is a bentonite treated with hydrophobic cationic materials. Other suitable clay suspending aids include colloidal magnesium aluminum silicates. Advantageously, the suspending aid is utilised at a level of from at least 1 %, and preferably at least 2% by weight of the base formulation, often at up to 8% and in some desirable embodiments at up to 6%. It is often beneficial to calculate the proportion of suspending aid by reference to the antiperspirant active, and in particular in a weight ratio of antiperspirant active : suspending aid of up to 15:1. Said ratio is greater than 4:1 in many embodiments, and herein a particularly desirable ratio range is from 6:1 to 12:1.

Herein, it is advantageous to employ an activator for the clay suspending aid, for example to encourage variations in the manufacturing process from the base formulation. Clay activators include ethanol and especially propylene carbonate. The amount of activator is preferably selected in the range of from 25 to 75% of the weight of the suspending aid, is advantageously at least 35% and is often not more than 60%. Accordingly, herein, in some advantageous compositions, the base formulation comprises from 1 to 3% by weight propylene carbonate. It has been observed that the addition of the activator can further enhance the ability of the clay-suspending aid-containing composition after topical application to offer observed smoothness and/or cover imperfections and/or improve the tone and/or the radiance of the skin.

Accordingly, in a further aspect of the present invention, the base formulation comprises in addition to the particulate aluminium antiperspirant salt, the oil and the clay suspending aid, (as described herein), a smoothing aid (i.e. wax and mica) as described herein together with the propylene carbonate.

The base formulation can comprise one or more optional constituents which have hitherto been incorporated or proposed for incorporation in anhydrous aerosol antiperspirant compositions. Such optional constituents may be liquid (in which event they are desirably miscible with the oils therein) or solid, and normally comprise in total not more than 10% and often not more than 5% by weight of the base formulation. Such optional constituents can comprise non-antiperspirant deodorant actives, such as antimicrobial actives such as polyhexamethylene biguanides, e.g. those available under the trade name Cosmocil™ or chlorinated aromatics, eg triclosan available under the trade name Irgasan™, non-microbiocidal deodorant actives such as triethylcitrate, bactericides and bacteriostats. Yet other deodorant actives can include zinc salts such as zinc ricinoleate. The compositions can additionally or alternatively contain, as a bacteristat, an iron chelator such as pentenoic acid which hinders bacterial growth/reproduction. The proportion of the deodorant active in the base formulation is often selected in the range of from about 0.05 to 2% by weight of the base formulation and especially from 0.1 to 0.5%.

Other optional ingredients can include sensory modifiers, such as talc or finely divided high molecular weight insoluble polyethylene, such as in an amount of up to 3% by weight; skin cooling agents such as menthol often selected in an amount of up 0.5%, particularly up to 0.2% of the base formulation and wash-off agents such as non-ionic surfactants, and particularly polyethoxylated fatty alcohols or acids, for example in an amount of up to about 3% of the base formulation. A further optional ingredient comprises a hair growth inhibitor, for example palmatine, such as at a concentration in the base formulation of up to 0.2% by weight, e.g. at least 0.01 %.

The base formulation desirably contains a fragrance, typically selected for its aesthetic appeal, such as at a concentration in the base formulation of from 0.1 to 8% by weight, and often at least 1 % such as from 2 to 6% by weight therein. A fraction of the fragrance, such as in the range of from 1 to 100%, particularly 25 to 75% can be encapsulated, for example in modified starch, or can be absorbed on and releasable from a cyclodextrin and/or can comprise a pro-fragrance.

### Aerosol compositions

### Propellant

The anhydrous aerosol composition herein comprises a propellant in addition to the base formulation described hereinabove. Commonly, the propellant is employed in a weight ratio to the base formulation of from 95:5 to 5:95, the weight proportion chosen depending in practice on the choice of propellants, the internal pressure which the manufacturer seeks to generate and the architecture of the aerosol canister. In such aerosol compositions the ratio of propellant to base formulation is from 70:30 to 90:10. Particularly suitable weight ratios of propellant:base formulation are in the regions of 3:1, 4:1 or 7:1, for example the propellant proportion of the total composition being within +/- 1% or 1.5% of 75%, 80% or 87% respectively.

Propellants herein generally accord with one of three classes; i) low boiling point gasses liquifided by compression, ii) volatile ethers and iii) compressed non-oxidising gases.

Class i) is conveniently a low boiling point material, typically boiling below-5°C, and often below-15°C, and in particular, alkanes and/or halogenated hydrocarbons. This class of propellant is usually liquefied at the pressure in the aerosol canister and evaporates to generate the pressure to expel the composition out of the canister. Examples of suitable alkanes include particularly propane, butane or isobutane, often in varying admixtures of the three components, and possibly containing a fraction of pentane or isopentane. Examples of halogenated hydrocarbons are fluorocarbons and chlorofluorocarbons such as, for example, 1,1-difluoroethane, 1-trifluoro-2-fluoroethane, dichlorodifluoromethane, 1-chloro-1,1-difluoroethane, and 1,1-dichloro-1,1,2,2-tetrafluoroethane. It is particularly desirable to employ the first class of propellant. Such propellants are commonly employed at a weight ratio to the base formulation of from 1:2 and especially at least 1:1 up to 95:5.

The second class of propellant comprises a very volatile ether of which the most widely employed ether hitherto is dimethyl ether. This propellant can advantageously be employed at relatively low weight ratio of propellant to base formulation, for example to as low as 5:95. It can also be employed in admixture with, for example, compressible/liquefiable alkane gasses.

The third class of propellant comprises compressed non-oxidising gasses, and in particular carbon dioxide or nitrogen. Inert gases like neon are a theoretical alternative.

### Aerosol Product Manufacture

An aerosol product according to the present invention can be made conveniently by
first blending together the ingredients of the base formulation in a vessel, less any ingredient intended to be added later,
agitating the mixture to suspend the particulate antiperspirant active,
charging an aerosol canister with the mixed base formulation and separately charging any other ingredient not blended previously into the base formulation,
charging either before, after or simultaneously,
fitting and sealing the discharge line containing the valve onto the aerosol canister and
injecting propellant gas into the canister through the discharge line.

By employing suitably intense mixing apparatus, such as by the use of a Sonolator or a Silverson mixer, at least some localised melting and/or dissolution of the wax ingredient can be effected, and particularly in respect of a wax having a comparatively low melting point such as up to 70°C.

The selection of the aerosol canister is at the discretion of the manufacturer of the aerosol product. Conveniently, the canister itself can be made from tin plate or aluminium. The discharge line includes a valve biased to the closed position, and may be depressed or tilt valve. The valve can be opened by depression or lateral movement as is determined by the valve of an actuator.

The discharge line terminates in a spray outlet, typically comprising a nozzle which in conventional aerosol dispensers is moulded with the spray nozzle to form a depressor button or which in more recent developments such as in EP 1040055 or EP 1255682 comprises rotational or lateral relative motion of elements to prevent the valve being opened prematurely. The nozzle outlet has an internal diameter that is usually selected within the range of from 300 to 800 microns, particularly not greater than 600 microns and in many embodiments from 350 to 450 microns. This is particularly beneficial in that it enables the composition to be sprayed without an undue risk of blockage through nozzles of similar internal diameter currently employable with corresponding otherwise similar compositions that lack the smoothing aid. This of course has the added advantage of allowing the canister to deliver a similar spray pattern, if desired, at a similar pressure to that of the smoothing aid-free composition.

According to a further aspect of the present invention there is provided an aerosol product according to claim 10. The particulate antiperspirant active may have >99% by weight of particles <75 microns and optionally contain a moisturiser and/or a triglyceride oil, disposed within an aerosol canister having a nozzle outlet of from 350 to 450 microns.

### Topical application

The aerosol composition of the instant invention can be sprayed onto skin, and particularly into the underarm (axilla) in a conventional manner for spraying liquid compositions. Very conveniently, the base formulation can be mixed with a propellant prior to being sprayed from an aerosol canister. The canister is desirably held at a distance of between 12 and 18 cms from the armpit and the valve in the discharge line opened. The composition can be sprayed at the discretion of the user for a conventional period of time, such as from at least about 1 or 1.5 seconds and preferably from about 2 to 5 seconds per armpit. The spray deposits the droplets of mainly base formulation onto the skin surface, which tend to spread and coalesce, forming a film, from which the volatile oils evaporate. When present, globules, such as globules of the wax, and particulates, such as the mica, and especially the mica pigment, including the mica interference pigment are exposed through the film and create the desired beneficial physical appearance.

Herein, except where the context demands otherwise, a numerical quantity, such as boundary or a ratio, can be qualified by the term "about".

Having described the invention, as well as preferred embodiments thereof, in general terms, particular embodiments thereof will described in more detail hereinafter by way of example only.

### Comparison CA and Examples 1 to 4

In this Comparison and Examples, a base formulation as prepared having the composition summarised in Table 1 below by charging a vessel with the oils, and thereafter slowly introducing the particulate materials with energetic propeller mixing employing a Silverson mixer, including any smoothing aid (wax flakes and/or mica). The mixer blades tended to promote localised heating resulting in at least partial melting of the wax. When the formulation was visually homogenous, the fragrance oil was blended in and the entire composition was charged into metal 150g canisters. A discharge line was inserted through the mouth of the canister and a valve embedded in the line was mounted in a valve cup crimped over the open mouth of the canister. Thereafter, the canister was charged with the propellant through the discharge line, in a weight ratio of 13 parts base formulation to 87 parts propellant. The propellant comprised a mixture of propane, butane and isobutane (CAP 40 ex Calor). An actuator was fitted on to the canister having a spray outlet orifice (nozzle orifice) with an internal diameter of 400 microns.

**Table 1**

| Comparison/Examples | CA | Ex 1 | Ex 2 | Ex 3 | Ex 4 |
|---|---|---|---|---|---|
| INCI name | % by weight | | | | |
| Cyclomethicone ¹ | 18.52 | 15.96 | 15.24 | 15.24 | 15.36 |
| Polydimethiconol in cyclopentasiloxane ² | 1.54 | 1.54 | 1.54 | 1.54 | 1.54 |
| PPG-14 Butyl Ether ³ | 22.24 | 22.24 | 22.24 | 22.24 | 22.24 |
| Octyldodecanol ⁴ | 0.90 | 0.90 | 0.90 | 0.90 | 0.90 |
| C12-15 Alkyl Benzoate ⁵ | 3.85 | 6.15 | 6.15 | 6.15 | 6.15 |
| Helianthus Annuus Seed Oil ⁶ | 4.00 | 4.00 | 4.00 | 4.00 | 4.00 |
| Disteardimonium Hectorite ⁷ | 4.23 | 3.85 | 3.85 | 3.85 | 3.85 |
| ButylHydroxyToluene | 0.77 | 0.77 | 0.77 | 0.77 | 0.77 |
| Fragrance | 5.38 | 5.38 | 5.38 | 5.38 | 5.38 |
| Propylene Carbonate | 0.12 | 0.00 | 0.12 | 0.12 | 0.00 |
| Stearyl Alcohol | 0.00 | 0.00 | 0.60 | 0.60 | 0.60 |
| Titanium Dioxide & mica ⁸ | 0.00 | 0.37 | 0.37 | 0.37 | 0.37 |
| Titanium Dioxide & mica ⁹ | 0.00 | 0.37 | 0.00 | 0.37 | 0.37 |
| Titanium Dioxide & mica ¹⁰ | 0.00 | 0.00 | 0.37 | 0.00 | 0.00 |
| Activated Aluminium Chlorohydrate Powder ¹¹ | 38.46 | 38.46 | 38.46 | 38.46 | 38.46 |

The ingredients were as follows:-

| | | |
|---|---|---|
| 1 | DC245 | Dow Corning |
| 2 | DC1501 | Dow Corning |
| 3 | Fluid AP | Amerchol |
| 4 | Eutanol G | Henkel |
| 5 | Finsolv TN | Fintex |
| 6 | AgriPure 80 | Cargill PLC |
| 7 | Bentone 38V | Elementis |
| 8 | Timiron MP-115 Ultra-luster | Merck |
| 9 | Timiron Super Blue | Merck |
| 10 | Timiron Silk Blue | Merck |
| 11 | Summit 7172 | Giulini |

The various compositions were tested in the following manner and the results summarised in Table 2 below:-
- For each pair of compositions, 10 Female panellists aged between 18 and 66 and in good health who did not have a history of skin disease and were not undergoing treatment for a skin condition, shaved and washed their own axillae approx 12hrs before the study commenced and refrained from applying antiperspirant or other cosmetic composition to the axillae afterwards. Their underarms were assessed by an expert clinician in a well lit booth of a clinical test centre immediately before topical application of the test compositions, randomly one of the pair to the left and the other to the right axilla;
- Skin quality was scored on a 0 to 5 scale for tone, imperfections, radiance, visual smoothness and visual and the result recorded;
- Test product (about 250-300mg) was applied to each axillae by the assessor with a 2 second spray in a ventilated booth;
- The Expert clinician assessment reassessed the skin quality attributes directly after product application on the same 0 to 5 scales in the same booth and the results recorded;
- The difference between the score before and after application of the test product for each attribute was calculated and the average from the panellists is summarised in Table 2 below.

Increases in tone, radiance and smoothness are recorded as positive as are decreases in imperfections, if recorded. Accordingly, the data summarised in Table 2 indicates the effectiveness of the Example compositions at improving the skin appearance and hence the perceived skin quality whilst applying a previously proven antiperspirant active.

The attributes were assessed by mapping onto the following scale (description of severity, coverage of axillae):-

| | |
|---|---|
| 0 | none |
| 1 | light, up to 10% |
| 2 | mild, 11 to 25% |
| 3 | moderate, 26 to 50% |
| 4 | marked, 51 to 75% |
| 5 | severe, 76 to 100% |

The assessment of the attributes was developed in conjunction with the test subjects and was made by considering inter-alia the following skin features:-
- Tone: unevenness, freckles, Hyperpigmentation through to evenness
- Radiance: dullness through healthy glow to brightness
- Smoothness: visible roughness, pits to velvety, peachy appearance.

For the avoidance of doubt, Example 1 is a Comparative Example.

**Table 2**

| Comparison/Examples | CA | Ex 1 | Ex 2 | Ex 3 | Ex 4 |
|---|---|---|---|---|---|
| Tone | 0.2 | 0.3 | 0.6 | 0.9 | 0.6 |
| Radiance | 0.35 | 0.6 | 0.6 | 0.8 | 0.9 |
| Smoothness | 0.3 | 0.4 | 0.6 | 0.9 | 1.0 |

From the data summarised in Table 2, it can be seen that the incorporation of at least one smoothing aid improved the perceived quality of the underarm skin compared with a comparison aerosol product that itself had previously been demonstrated to exhibit improved moisture retention in the skin relative its predecessor, but without offering significant perceivable improvement in underarm skin appearance (Comparison A). In particular, Example 1 shows that incorporation of even a low concentration of two mica pigments, including in particular an interference pigment exhibiting blue highlights improved not only the tone of the skin, but showed a greater improvement for both radiance and smoothness, indicating to the user a healthier and younger-looking skin. Examples 2, 3 and 4 show not only that incorporation of a wax improved the perception of the skin, but that the presence of both the wax and the mica pigment offered a greater benefit than the mica pigment alone.

## Claims

1. An anhydrous antiperspirant composition comprising a base formulation and a propellant having a boiling point at standard pressure of below 10°C, which formulation comprises a particulate astringent aluminium salt, an oil and optionally a particulate clay or silica suspending aid, and which further comprises a smoothing aid comprising a wax having a melting point of from 50°C to 90°C contained at from 0.4 to 1.5% by weight of the base composition, the wax comprising stearyl alcohol, and mica at at feast 0.1% by weight of the base composition, wherein the propellant and the base formulation are present in a weight ratio of from 9:1 to 7:3.

2. A composition according to any of the preceding claims in which the mica further comprises a coating with titanium dioxide tin oxide and/or silicon dioxide forming a mica pigment.

3. A composition according to any preceding claim in which the mica has a weight average mean particle size in the range of from 7 to 30 µm.

4. A composition according to any preceding claim in which the mica is a mixture of two materials, one material having a weight average mean particle size in the range of from 18 to 25 µm and the other material having a weight average mean particle size in the range of from 7 to 14 µm, and at least one of which is a mica pigment.

5. A composition according to any preceding claim in which the mica comprises one or more interference pigments having silver, green, violet and/or blue highlights.

6. A composition according to any preceding claim in which the astringent antiperspirant is present in a weight ratio to the smoothing aid selected in the range of from 12.5:1 to 50:1.

7. A composition according to any preceding claim in which the astringent antiperspirant salt comprises an aluminium chlorohydrate having a weight average mean particle size (D50) in the range of from 15 to 30µm and >99% by weight of its particles of below 100µm and preferably below 80µm diameter.

8. A composition according to any preceding claim in which the base formulation comprises a hectorite clay, optionally hydrophobically surface treated and further comprises propylene carbonate.

9. A composition according to any preceding claim in which the base formulation contains from 1 to 8% by weight of a triglyceride oil.

10. An aerosol product comprising an aerosol composition according to any preceding claim charged into a dispensing canister comprising a reservoir, a spray outlet, a passageway for fluid leading from the reservoir to the spray outlet including a valve and an actuator for opening and closing the valve.

11. A product according to claim 10 in which the spray outlet has an internal diameter of from 350 to 600 microns and preferably from 350 to 450 microns.

12. A method for reducing perspiration comprising topically applying to skin a composition according to any preceding claim and particularly in the underarm.

## Patentansprüche

1. Wasserfreie schweißhemmende Zusammensetzung, die eine Basisformulierung und ein Treibmittel mit einem Siedepunkt bei Standarddruck von unter 10°C umfasst, wobei die Formulierung ein teilchenförmiges adstringierendes Aluminiumsalz, ein Öl und optional eine teilchenförmige Ton- oder Siliziumdioxid-Suspendierhilfe umfasst und welche ferner eine Glättungshilfe umfasst, umfassend ein Wachs mit einem Schmelzpunkt von 50°C bis 90°C, enthalten in einer Menge von 0,4 bis 1,5%, bezogen auf das Gewicht der Basiszusammensetzung, wobei das Wachs Stearylalkohol und Glimmer in mindestens 0,1%, bezogen auf das Gewicht der Basiszusammensetzung, umfasst, wobei das Treibmittel und die Basisformulierung in einem Gewichtsverhältnis von 9:1 bis 7:3 vorliegen.

2. Zusammensetzung nach irgendeinem der vorhergehenden Ansprüche, in welcher der Glimmer ferner eine Beschichtung mit Titandioxid, Zinnoxid und/oder Siliziumdioxid umfasst, um ein Glimmer-Pigment zu bilden.

3. Zusammensetzung nach irgendeinem vorhergehenden Anspruch, in welcher der Glimmer eine gewichtsgemittelte durchschnittliche Partikelgröße in dem Bereich von 7 bis 30 µm aufweist.

4. Zusammensetzung nach irgendeinem vorhergehenden Anspruch, in welcher der Glimmer eine Mischung von zwei Materialien darstellt, wobei ein Material eine gewichtsgemittelte durchschnittliche Partikelgröße in dem Bereich von 18 bis 25 µm und das andere Material eine gewichtsgemittelte durchschnittliche Partikelgröße in dem Bereich von 7 bis 14 µm aufweist und mindestens eines hiervon ein Glimmer-Pigment darstellt.

5. Zusammensetzung nach irgendeinem vorhergehenden Anspruch, in welcher der Glimmer ein oder mehrere Interferenzpigmente mit Silber-, Grün-, Violett- und/oder Blau-Highlights umfasst.

6. Zusammensetzung nach irgendeinem vorhergehenden Anspruch, in welcher das adstringierende schweißhemmende Mittel zu der Glättungshilfe in einem Gewichtsverhältnis vorliegt, das in dem Bereich von 12,5:1 bis 50:1 ausgewählt ist.

7. Zusammensetzung nach irgendeinem vorhergehenden Anspruch, in welcher das adstringierende schweißhemmende Salz ein Aluminiumchlorhydrat umfasst, das eine gewichtsgemittelte durchschnittliche Partikelgröße (D50) in dem Bereich von 15 bis 30 µm aufweist und > 99 Gewichts-% seiner Partikel einen Durchmesser unter 100 µm und bevorzugt unter 80 µm aufweisen.

8. Zusammensetzung nach irgendeinem vorhergehenden Anspruch, in welcher die Basisformulierung einen Hectorit-Ton, optional hydrophob oberflächenbehandelt, und ferner Propylencarbonat umfasst.

9. Zusammensetzung nach irgendeinem vorhergehenden Anspruch, in welcher die Basisformulierung 1 bis 8 Gewichts-% eines Triglyceridöls enthält.

10. Aerosol-Produkt, das eine Aerosol-Zusammensetzung nach irgendeinem vorhergehenden Anspruch umfasst, die in einen Dosierkanister gefüllt ist, umfassend ein Reservoir, einen Sprühauslass, einen Durchgang für die Flüssigkeit, der von dem Reservoir zu dem Sprühauslass führt, der ein Ventil und einen Stellantrieb zum Öffnen und Verschließen des Ventils einschließt.

11. Produkt nach Anspruch 10, in welchem der Sprühauslass einen Innendurchmesser von 350 bis 600 Mikron und bevorzugt von 350 bis 450 Mikron aufweist.

12. Verfahren zur Verminderung von Schweiß, das das topische Aufbringen einer Zusammensetzung nach irgendeinem vorhergehenden Anspruch auf die Haut und insbesondere in den Bereich des Unterarms umfasst.

## Revendications

1. Composition d'antiperspirant anhydre comprenant une formulation de base et un propulseur ayant un point d'ébullition à pression atmosphérique inférieur à 10°C, laquelle formulation comprend un sel d'aluminium astringent particulaire, une huile et éventuellement un auxiliaire de mise en suspension de silice ou d'argile particulaire, et laquelle comprend de plus un auxiliaire de lissage comprenant une cire ayant un point de fusion de 50°C à 90°C contenu à de 0,4 à 1,5 % en masse de la composition de base, la cire comprenant de l'alcool stéarylique, et du mica à au moins 0,1 % en masse de la composition de base, où le propulseur et la formulation de base sont présents dans un rapport massique de 9:1 à 7:3.

2. Composition selon l'une quelconque des revendications précédentes, dans laquelle le mica comprend de plus un revêtement avec du dioxyde de titane oxyde d'étain et/ou dioxyde de silicium formant un pigment de mica.

3. Composition selon l'une quelconque des revendications précédentes où le mica présente une taille moyenne de particule moyenne en masse dans l'intervalle de 7 à 30 µm.

4. Composition selon l'une quelconque des revendications précédentes, dans laquelle le mica est un mélange de deux matériaux, un matériau ayant une taille moyenne de particule moyenne en masse dans l'intervalle de 18 à 25 µm et l'autre matériau ayant une taille moyenne de particule moyenne en masse dans l'intervalle de 7 à 14 µm, et dont au moins un est un pigment de mica.

5. Composition selon l'une quelconque des revendications précédentes, dans laquelle le mica comprend un ou plusieurs pigments d'interférence présentant des brillances d'argent, de vert, de violet et/ou de bleu.

6. Composition selon l'une quelconque des revendications précédentes, dans laquelle l'antiperspirant astringent est présent dans un rapport massique à l'auxiliaire de lissage choisi dans l'intervalle de 12,5:1 à 50:1.

7. Composition selon l'une quelconque des revendications précédentes, dans laquelle le sel d'antiperspirant astringent comprend un chlorohydrate d'aluminium ayant une taille moyenne de particule moyenne en masse (D50) dans l'intervalle de 15 à 30 µm et > 99 % en masse de ses particules de diamètre inférieur à 100 µm et de préférence inférieur à 80 µm.

8. Composition selon l'une quelconque des revendications précédentes, dans laquelle la formulation de base comprend une argile d'hectorite, éventuellement hydrophobiquement traitée en surface et comprend de plus du carbonate de propylène.

9. Composition selon l'une quelconque des revendications précédentes, dans laquelle la formulation de base contient de 1 à 8 % en masse d'une huile de triglycéride.

10. Produit d'aérosol comprenant une composition d'aérosol selon l'une quelconque des revendications précédentes chargée dans un réservoir de distribution comprenant un réservoir, une sortie de pulvérisation, un passage pour mener du fluide du réservoir jusqu'à la sortie de pulvérisation incluant une vanne et un dispositif d'actionnement pour ouvrir et fermer la vanne.

11. Produit selon la revendication 10, dans lequel la sortie de pulvérisation présente un diamètre interne de 350 à 600 microns et de préférence de 350 à 450 microns.

12. Procédé de réduction de la transpiration comprenant l'application topique à la peau d'une composition selon l'une quelconque des revendications précédentes et particulièrement dans l'aisselle.
